# FASCICULE DE BREVET EUROPEEN

(11) **EP 3 041 556 B1**
(45) Date de publication et mention de la délivrance du brevet: **30.12.2020**
(21) Numéro de dépôt: 14766902.2
(22) Date de dépôt: 05.09.2014
(51) Int. Cl.: A61M 16/00, A61M 16/06

(54) **EQUIPEMENT DE TRAITEMENT D'UN TROUBLE RESPIRATOIRE OBSTRUCTIF**
VORRICHTUNG ZUR BEHANDLUNG OBSTRUKTIVER ATMUNGSERKRANKUNGEN
EQUIPMENT FOR TREATING AN OBSTRUCTIVE RESPIRATORY DISORDER

(30) Priorité: 05.09.2013 FR 1302063
(43) Date de publication de la demande: 13.07.2016
(73) Titulaire: Physio-Assist, 13593 Aix-en-Provence Cedex 3 (FR)
(72) Inventeur: LANTZ, Jean-Sébastien, 13593 Aix-en-Provence Cedex 3 (FR); MITHALAL, Adrien, 13593 Aix-en-Provence Cedex 3 (FR)
(74) Mandataire: Icosa
(86) Numéro de dépôt international: PCT/EP2014/002405
(87) Numéro de publication internationale: WO 2015/032503

(56) Documents cités:
- EP-A1- 2 316 514
- EP-A2- 1 800 705
- DE-B3-102007 029 016
- FR-A1- 2 917 979
- US-A1- 2005 039 749
- US-A1- 2008 066 754
- US-A1- 2008 294 090
- US-A1- 2009 126 731
- US-B1- 6 632 194

## Description

La présente demande internationale revendique la priorité de la demande de brevet français FR 13/02063 déposée en date du 5 septembre 2013.

La présente invention concerne le domaine du traitement des troubles ventilatoires obstructifs.

Un patient atteint de trouble respiratoire obstructif, ou syndrome obstructif pulmonaire (ex., BPCO, mucoviscidose), présente une accumulation de mucus. Cette accumulation de mucus est la résultante d'une résistance de celui-ci aux mécanismes naturels de drainage bronchique et entraine une limitation des débits dans l'arbre bronchique.

Outre la gêne respiratoire, cette accumulation de mucus est susceptible d'entraîner de lourdes complications pulmonaires car sa stagnation constitue un terreau propice aux infections bactériennes.

Il convient donc dans le traitement des patients atteints de trouble respiratoire obstructif d'être particulièrement vigilent pour limiter ces risques de surinfection.

Quelques dispositifs ont été développés pour aider ces patients dans leur toilette bronchique (l'IPV® (Intrapulmonary Percussive Ventilation), AIR HELP, etc.).

Le document US 2009/126731 décrit un ensemble interface patient comprenant un logement définissant un port d'entrée et un port de sortie auquel est connecté le patient. Cette interface est connectée, via son port d'entrée, à un dispositif générant des impulsions avec une fréquence allant de 2 à 25 Hz et dans lequel une pompe génère un flux d'air sous pression. Un nébuliseur est avantageusement fluidiquement connecté au port de sortie et placé pour introduire un médicament dans le flot d'air et délivrer un air médicalisé au patient.

Le document US 2008/066754 décrit un dispositif respiratoire à oscillation continue haute fréquence délivrant un traitement tout à la fois pendant l'inhalation et l'expiration de manière à optimiser le nettoyage des sécrétions bronchiques. Un circuit venturi de l'interface patient est connecté avec un aérosol médicamenteux pout délivrer un traitement oscillatoire continu à haute fréquence.

Le document US 2005/039749 décrit un système d'inspiration-expiration pour le retrait des sécrétions broncho-pulmonaires avec un déclenchement automatique de la phase d'inspiration comprenant une conduite pour relier aux voies aériennes d'un patient, une source de pression avec un port à pression positive et un port à pression négative. Le système comprend un dispositif de commutation reliant sélectivement la conduite au port de pression positive, le port de pression négative à l'air libre, un capteur apte à détecter une inspiration par le patient et un système de contrôle pour piloter le dispositif de commutation pour relier les conduites séquentiellement au port positif, au port négatif, à l'air libre et revenir au port positif en réponse au capteur détectant une inspiration du patient lorsque la conduite est reliée à l'air libre.

Maintenant, ces dispositifs sont très peu utilisés, une des raisons associée à cette faible utilisation étant le risque de contamination, et notamment d'intercontamination, lié à l'utilisation du même appareil par plusieurs patients.

Le brevet DE 10 2007 029016 décrit un équipement respiratoire thérapeutique comprenant un embout, lequel embout est équipé d'un dispositif de codage interrogeable à distance et permettant l'activation de l'équipement respiratoire dès lors qu'il est identifié par ce dernier.

La demande de brevet EP 01800705 décrit lui des composants pour un appareil de ventilation d'un patient comprenant une étiquette de codage lisible à distance. Le contrôleur du générateur de flux est programmé pour recevoir une donnée issue de l'étiquette d'identification via un lecteur d'étiquette et adapter les fonctions du contrôleur de flux pour s'accorder avec le composant.

Les inventeurs ont maintenant développé une solution, susceptible de s'adapter à ces dispositifs, sans en altérer les performances, et de limiter au maximum les risques de contamination et notamment d'intercontamination.

Ainsi, la présente invention vise un équipement de traitement des troubles ventilatoires obstructifs comprenant:
- un embout respiratoire (2) comprenant au moins un moyen de filtration d'air (21) et au moins un transpondeur (22) sur lequel est enregistré un identifiant unique, et
- un dispositif (1) destiné à faciliter l'expectoration du mucus et comprenant au moins un récepteur (11) qui est associé à ce transpondeur (22) et qui ne permet la mise en route du dispositif qu'après la détection dudit identifiant unique,
caractérisé en ce que :
- ledit transpondeur (22) comprend en outre des moyens de stockage d'une information caractérisant l'utilisation de l'embout respiratoire, l'information correspondant au nombre d'utilisations de l'embout respiratoire (2); et
- ledit dispositif comprend au moins un microcontrôleur (12) destiné à la gestion du transpondeur (22), lequel est tout à la fois en charge de la lecture de l'information de ce transpondeur (22) que de la modification de celle-ci en vue d'empêcher la mise en route du dispositif (1) si le nombre d'utilisations de l'embout respiratoire (2) est supérieur à un seuil prédéterminé.

Au-delà de sa définition « classique », l'expression «troubles ventilatoires obstructifs » peut s'étendre ici aux troubles associés à l'obstruction des voies nasales, telle que la sinusite, et présentant la même problématique. La sinusite correspond à l'obstruction des sinus consécutive à une inflammation des muqueuses du nez résultant en un drainage altéré du mucus nasal ; dans lequel mucus vont alors se développer rapidement des bactéries.

Des « dispositifs (1) destinés à faciliter l'expulsion de mucus », de préférence destiné à faciliter l'expectoration de mucus, sont bien connus de l'homme du métier et incluent notamment les dispositifs décrits dans le brevet US 5,862,802 et dans le brevet français FR 2,733,917.

Un « embout respiratoire » (2) correspond aussi bien à un masque respiratoire, à un embout buccal, qu'à un embout nasal. Maintenant, un tel embout respiratoire sera plus généralement un masque respiratoire ou un embout buccal.

Pour ce qui est des moyen de filtration d'air (21) susceptibles d'être associées à un tel embout respiratoire (2); il sont largement connus aujourd'hui et correspondent, le plus souvent, à des membranes de filtration. De telles membranes de filtration sont notamment commercialisées par la société MILLIPORE®.

En lien avec le transpondeur (22), qui est un élément critique de l'invention, il peut prendre la forme d'une étiquette radiofréquence ou support RFID. Une telle étiquette (support) comprend de façon connue une boucle d'induction assurant l'alimentation d'un circuit comprenant une mémoire dans laquelle est enregistré un identifiant unique. Cette étiquette radiofréquence ou support RFID est destiné à transmettre au dispositif (1) un identifiant unique de sorte d'éviter son utilisation par plusieurs patients et la transmission d'infection entre eux. A titre d'exemple, une telle étiquette radiofréquence peut fonctionner dans la bande de fréquence comprise entre 13 et 14 MHz.

Dans un exemple non-revendiqué et qui ne fait pas parti de la présente invention, le transpondeur (22) comprendra des moyens de stockage d'une information caractérisant l'utilisation de l'embout respiratoire (2), l'information correspondant à l'un des états suivants :
- Non utilisé
   ou
- Utilisé.

L'information « Non utilisé » permet la mise en route du dispositif destiné à faciliter l'expulsion du mucus, alors que l'information « Utilisé » ne le permet pas.

Maintenant et dans le cas où l'équipement selon l'invention serait au domicile même du patient par exemple, on pourra imaginer un transpondeur (22) permettant une utilisation plus longue de l'embout respiratoire (2). Dans un tel cas, l'information stockée sur le transpondeur (22) correspondra non plus à deux états, mais au nombre d'utilisations de l'embout respiratoire (Utilisé 1 fois, Utilisé 2 fois, ..., Utilisé n fois), et le dispositif (1) associé ne se mettra en en route que si le nombre d'utilisations est inférieur à un seuil prédéterminé ; puis il inscrira, au terme de chaque cycle, une utilisation supplémentaire sur le transpondeur (22), de préférence le support RFID.

Outre ces informations, le transpondeur (22) pourra également mémoriser, en vue de leur transmission au dispositif (1), des paramètres relatifs à l'embout respiratoire (date de fabrication, date de péremption, numéro de lot, etc.) ou des paramètres relatifs au fonctionnement du dispositif (1) (fréquence, puissance, durée, etc.). De la sorte, il est possible d'associer un transpondeur (22), et donc un embout respiratoire (2), à un type de patient donné (BPCO, sinusite, nouveau né, etc.) de sorte que le dispositif (1), mis en présence de celui-ci, adapte automatiquement les paramètres à ce type de patient et optimise ainsi l'expulsion de mucus par ce patient.

En face, le dispositif (1) est muni d'une alimentation qui permet d'activer le transpondeur (22) (ex. l'étiquette radiofréquence ou support RFID). Dans le cas d'une étiquette radiofréquence ou support RFID, cette activation va s'opérer par le biais d'un champ magnétique capté par la boucle d'induction du support RFID. Le récepteur du dispositif est alors une antenne destinée à recevoir les signaux électromagnétiques émis par l'étiquette radiofréquence ou support RFID lorsque celle-ci est alimentée. L'activation du dispositif (1) est conditionnée à la détection d'un signal d'identification du transpondeur (22). En l'absence d'un tel signal, le dispositif (1) est maintenu en position de veille pour éviter, à tout le moins pour limiter, le risque de transmission d'une infection d'un patient à un autre du fait de l'utilisation d'un même embout respiratoire (2).

Maintenant, l'équipement selon l'invention pourra comprendre d'autres « consommables » associés eux aussi à un transpondeur de sorte d'éviter tout risque de transmission de germes à un patient.

On pourra ainsi imaginer un autre moyen de filtration de l'air, directement en sortie du dispositif (1) et constituant une seconde sécurité ; lequel comprend un autre transpondeur organisant le changement de celui-ci, par exemple tout les mois ou toutes les 100 utilisations.

On pourra encore imaginer un tuyau flexible entre le dispositif (1) et l'embout respiratoire (2), lui aussi comprenant un troisième transpondeur; ce dernier transpondeur imposant le changement du flexible toutes les semaines ou toutes les 25 utilisations.

Un procédé de traitement d'un trouble ventilatoire obstructif est également décrit. Ce procédé comprend les étapes suivantes :
- Associer un embout respiratoire (2) au dispositif (1)
- Approcher l'embout respiratoire (2) du dispositif (1) pour que le dispositif de lecture puisse lire les informations du support transpondeur (22),
- Initier le traitement.

L'invention porte également sur l'utilisation d'un microcontrôleur (12) d'un dispositif (1) destiné à faciliter l'expectoration du mucus pour la gestion d'un transpondeur (22) d'un embout respiratoire (2), lequel transpondeur (22) sur lequel est enregistré un identifiant unique est associé à au moins un récepteur (11) dudit dispositif, lequel microcontrôleur (12)
(i) ne permet la mise en route du dispositif qu'après la détection dudit identifiant unique; et
(ii) permet la lecture et la modification de l'information de ce transpondeur (22) correspondant au nombre d'utilisations de l'embout respiratoire (2) en vue d'empêcher la mise en route du dispositif (1) si le nombre d'utilisations de l'embout respiratoire (2) est supérieur à un seuil prédéterminé.

D'autres caractéristiques, buts et avantages de la présente invention apparaîtront à la lecture de la description détaillée qui va suivre, au regard des dessins annexés, donnés à titre d'exemples non limitatifs et sur lesquels:

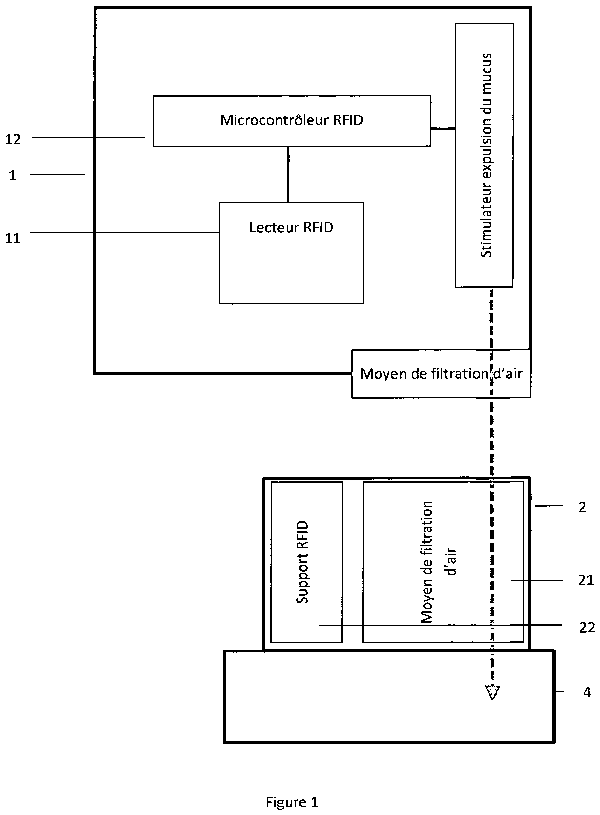

La figure 1 est un diagramme schématique d'un équipement selon l'invention.

L'invention repose sur l'intégration d'un transpondeur (22) à l'embout respiratoire (2) de sorte de rendre accessible au plus grand nombre les dispositifs destinés à faciliter l'expulsion de mucus en évitant, ou tout du moins en limitant, les risques d'infection et notamment de transmission d'infection entre les patients utilisant un même appareil.

En référence à la figure 1, le dispositif de traitement (1) comprend un lecteur RFID (11) (sous forme d'antenne RFID par exemple) permettant de sécuriser l'ensemble du dispositif en ne permettant la mise en route de celui-ci qu'après avoir détecté un support RFID (22) associé à un embout respiratoire (2), évitant ainsi tout risque d'infection pour le patient (4) à traiter ; et de sécuriser le traitement en lisant les paramètres préenregistrés dans le support (22), ne permettant pas à un autre patient d'utiliser par la suite le même embout respiratoire (2). Le dispositif de traitement (1) comprend également un microcontrôleur (12) responsable de la gestion du dispositif (1) et de l'embout respiratoire (2).

L'embout respiratoire (2) est en contact avec le patient (4). De ce fait, il doit répondre aux problématiques de stérilité, critiques avec ce type de patients (p.ex. mucoviscidose) et c'est à ce titre que celui-ci comprend un moyen de filtration de l'air (21). L'embout respiratoire (2) comprend également un support RFID (22) ; lesquels supports RFID sont largement décrits dans l'état de l'art. Cependant, la présente divulgation prévoit aussi que le support RFID (22) puisse également avoir pour fonction d'automatiser le procédé de traitement. L'utilisateur pourra ainsi en éventuellement sélectionner le support RFID en fonction du patient à traiter et le support sélectionné délivrera au récepteur un signal ou une information qui pilotera le dispositif (puissance, fréquence, etc. en fonction de la pathologie et/ou du patient à traiter). Dans ce cadre, le support RFID possèdera un identifiant qui renverra vers une table de paramétrage localisée dans le logiciel du dispositif dans laquelle sont prédéfinies la durée, la fréquence et/ou encore la puissance de stimulation, mais aussi éventuellement le type de patient qui doit être traité (pour une vérification de sécurité de la part du praticien par exemple). Lors de l'utilisation du dispositif, l'utilisateur n'a qu'à choisir l'embout respiratoire adapté (qui contient les supports RFID) en fonction du patient (p. ex. adulte ou nourrisson) et de l'indication (p.ex. BPCO ou sinusite). De la sorte, aucun ajustage ou presque des paramètres par l'utilisateur n'est nécessaire. Pour éviter encore les risques de contamination, un processus de stérilisation de l'embout respiratoire (2), notamment à l'oxyde d'éthylène, peut être prévu.

En référence à la figure 1 toujours, la présence d'un support RFID (22) est indiquée au microcontrôleur 12. La présence d'un support RFID (22) ne donne pas d'information concernant son état. Pour connaître l'état du support RFID (22), le microcontrôleur commande une lecture du support RFID (22). Cette commande permet de connaître l'état d'utilisation du support RFID (22) présent dans le champ du lecteur RFID (11). Dans un exemple non-revendiqué et qui ne fait pas parti de la présente invention, les différents états d'un support RFID (22) sont :
- Utilisé
- Non Utilisé

Il peut en outre comprendre d'autres informations relatives au paramétrage du dispositif.

Le microcontrôleur (12) demande l'état du support RFID (22) et, éventuellement, les paramètres de stimulation du mucus. En fonction de la réponse, le début de la stimulation autorisé et les paramètres de celle-ci ajustée au besoin. Simultanément, les informations du support RFID (22) (Utilisé) sont mises à jour.

Dans un certain cadre d'utilisation, l'ensemble RFID et embout respiratoire servent également à réaliser une étude clinique dit en « double aveugle ». En plus de contenir les paramètres du dispositif, l'embout respiratoire contient une information stipulant si le dispositif doit effectuer une stimulation du mucus réelle ou simplement la simuler. Il est prévu que le dispositif destiné à faciliter l'expulsion de mucus, lorsque ce dernier stimule réellement ou pas le mucus ait le même comportement perceptible par le patient. La programmation des embouts est effectuée lors de leur fabrication par un logiciel qui aléatoirement autorise ou non la stimulation « réelle » à travers les paramètres contenus dans le support RFID de l'embout respiratoire.

Ce même logiciel permet de lire les informations concernant l'autorisation de stimulation du mucus. Le praticien traite alors le patient à l'aide de l'embout respiratoire sans savoir si la stimulation est réellement délivrée par le dispositif. Après utilisation de l'embout respiratoire, le praticien remet l'embout respiratoire à une personne chargée de l'étude clinique avec l'appréciation qu'il fait du résultat de la stimulation. La personne chargée de l'étude lit alors l'embout respiratoire afin de déterminer si le patient a été stimulé ou non.

Ainsi, avec un tel procédé, aucun intervenant direct ne peut savoir quand le dispositif effectue la stimulation réellement ou non durant l'essai clinique. Ce principe permet de recueillir des avis sur le résultat de la stimulation d'une complète objectivité.

Bien évidemment, l'ensemble des valeurs numériques de la description ne sont données qu'à titre purement indicatif afin d'orienter la réalisation de l'invention. On pourra donc utiliser d'autres valeurs numériques, par exemple déterminées par expérimentation, sans sortir du cadre de l'invention.

## Revendications

1. Un équipement de traitement des troubles ventilatoires obstructifs comprenant :
• un embout respiratoire (2) comprenant au moins un moyen de filtration d'air (21) et au moins un transpondeur (22) sur lequel est enregistré un identifiant unique, et
• un dispositif (1) destiné à faciliter l'expectoration du mucus et comprenant au moins un récepteur (11) qui est associé à ce transpondeur (22) et qui ne permet la mise en route du dispositif qu'après la détection dudit identifiant unique, **caractérisé en ce que :**
• ledit transpondeur (22) comprend en outre des moyens de stockage d'une information caractérisant l'utilisation de l'embout respiratoire, l'information correspondant au nombre d'utilisations de l'embout respiratoire (2); et
• ledit dispositif comprend au moins un microcontrôleur (12) destiné à la gestion du transpondeur (22), lequel est tout à la fois en charge de la lecture de l'information de ce transpondeur (22), que de la modification de celle-ci en vue d'empêcher la mise en route du dispositif (1) si le nombre d'utilisations de l'embout respiratoire (2) est supérieur à un seuil prédéterminé.

2. L'équipement selon la revendication 1, **caractérisé en ce que** le dispositif (1) inscrit, au terme de chaque cycle, une utilisation supplémentaire sur le transpondeur (22).

3. L'équipement selon l'une quelconque des revendications précédentes, **caractérisé en ce que le** transpondeur (22) est associé à un type de patient donné de sorte que le dispositif (1) mis en présence de celui-ci, adapte automatiquement les paramètres à ce type de patient.

4. L'équipement selon l'une quelconque des revendications précédentes, **caractérisé en ce que** ledit embout respiratoire (2) est choisi dans le groupe comprenant les masques respiratoires, les embouts buccaux et les embouts nasaux.

5. L'équipement selon l'une quelconque des revendications précédentes, **caractérisé en ce que** ledit transpondeur (22) est une étiquette radiofréquence ou un support RFID.

6. Une utilisation d'un microcontrôleur (12) d'un dispositif (1) destiné à faciliter l'expectoration du mucus pour la gestion d'un transpondeur (22) d'un embout respiratoire (2), lequel transpondeur (22) sur lequel est enregistré un identifiant unique est associé à au moins un récepteur (11) dudit dispositif, lequel microcontrôleur (12)
(i) ne permet la mise en route du dispositif qu'après la détection dudit identifiant unique; et
(ii) permet la lecture et la modification de l'information de ce transpondeur (22) correspondant au nombre d'utilisations de l'embout respiratoire (2) en vue d'empêcher la mise en route du dispositif (1) si le nombre d'utilisations de l'embout respiratoire (2) est supérieur à un seuil prédéterminé.

## Patentansprüche

1. Ausrüstung zur Behandlung obstruktiver Atemwegserkrankungen, umfassend:
• ein Atemmundstück (2), das mindestens ein Luftfiltermittel (21) und mindestens einen Transponder (22) umfasst, auf dem eine individuelle Kennung verzeichnet ist, und
• eine Vorrichtung (1), die zur Erleichterung des Auswerfens des Schleims bestimmt ist und mindestens einen Empfänger (11) umfasst, der diesen Transponder (22) zugeordnet ist und der das Ingangsetzen der Vorrichtung erst nach Ermittlung der individuellen Kennung gestattet,
**dadurch gekennzeichnet, dass**:
• der Transponder (22) ferner Speichermittel einer Information umfasst, die die Verwendung des Atemmundstücks kennzeichnet, wobei die Information der Anzahl der Verwendungen des Atemmundstücks (2) entspricht; und
• die Vorrichtung mindestens einen Microcontroller (12) umfasst, der zur Verwaltung des Transponders (22) bestimmt ist, der sowohl für das Lesen der Information dieses Transponders (22) als auch für das Ändern derselben im Hinblick auf die Verhinderung des Ingangsetzens der Vorrichtung (1) zuständig ist, wenn die Anzahl der Verwendungen des Atemmundstücks (2) über einem vorher festgelegten Grenzwert liegt.

2. Ausrüstung nach Anspruch **1**, **dadurch gekennzeichnet, dass** die Vorrichtung (1) am Ende jedes Zyklus eine weitere Verwendung in den Transponder (22) einträgt.

3. Ausrüstung nach einem der vorangehenden Ansprüche, **dadurch gekennzeichnet, dass** der Transponder (22) einem bestimmten Patiententypen derart zugeordnet ist, dass die in Anwesenheit desselben verbrachte Vorrichtung (1) die Parameter automatisch an diesen Patiententypen anpasst.

4. Ausrüstung nach eine der vorangehenden Ansprüche, **dadurch gekennzeichnet, dass** das Atemmundstück (2) aus der Gruppe ausgewählt ist, die die Atemmasken, die Mundstücke und die Nasenstücke umfasst.

5. Ausrüstung nach einem der vorangehenden Ansprüche, **dadurch gekennzeichnet, dass** der Transponder (22) ein Hochfrequenzetikett oder ein RFID-Träger ist.

6. Verwendung eines Microcontrollers (12) einer Vorrichtung (1), die zur Erleichterung des Auswerfens des Schleims bestimmt ist, für die Verwaltung eines Transponders (22) eines Atemmundstücks (2), wobei der Transponder (22), auf dem eine individuelle Kennung verzeichnet ist, mindestens einem Empfänger (11) der Vorrichtung zugeordnet ist, wobei der Mikrocontroller (12)
(i) das Ingangsetzen der Vorrichtung erst nach Ermittlung der individuellen Kennung erlaubt; und
(ii) das Lesen und das Ändern der Information dieses Transponders (22), die der Anzahl der Verwendungen des Atemmundstücks (2) entspricht, im Hinblick auf die Verhinderung des Ingangsetzens der Vorrichtung (1) gestattet, wenn die Anzahl der Verwendungen des Atemmundstücks (2) über einem vorher festgelegten Grenzwert liegt.

## Claims

1. Equipment for treating obstructive lung disorders including:
• a respiratory nozzle (2) comprising at least one air filtering means (21) and at least one transponder (22) on which is recorded a unique identifier, and
• a device (1) for facilitating the expectoration of mucus and comprising at least one receiver (11) which is associated with this transponder (22) and which only allows starting of the device after detection of said unique identifier,
**characterised in that:**
• said transponder (22) further comprises means for storing information characterising the use of respiratory nozzle, said information corresponding to the number of uses of the respiratory nozzle (2); and
• said device comprises at least one microcontroller (12) for the management of the transponder (22), which is designed both for reading the information of this transponder (22) and for modifying said information so as to prevent the starting of the device (1) if the number of uses of respiratory nozzle (2) is greater than a predetermined threshold.

2. The equipment according to claim **1, characterised in that** said device (1) registers, at the end of each cycle, a supplementary use on the transponder (22).

3. The equipment according to any one of the preceding claims, **characterised in that** said transponder (22) is associated with a given type of patient so that the device (1) placed in the presence thereof, automatically adjusts the settings for this type of patient.

4. The equipment according to any one of the preceding claims, **characterised in that** said respiratory nozzle (2) is selected from the group consisting of respiratory masks, mouthpieces, and nasal inserts.

5. The equipment according to any one of the preceding claims, **characterised in that** said transponder (22) is a radio frequency tag or a RFID holder.

6. Use of a microcontroller (12) of a device (1) for facilitating the expectoration of mucus for the management of a transponder (22) of a respiratory nozzle (2), said transponder (22) on which is recorded a unique identifier is associated to at least one receiver (11) of said device, said microcontroller (12):
i. only allows starting of the device after detection of said unique identifier; and
ii. allows reading and modifying of an information on said transponder (22) corresponding to the the number of uses of the respiratory nozzle (2) so as to prevent the starting of the device (1) if the number of uses of respiratory nozzle (2) is greater than a predetermined threshold.
